# EUROPÄISCHE PATENTSCHRIFT

(11) **EP 2 092 061 B1**
(45) Veröffentlichungstag und Bekanntmachung des Hinweises auf die Patenterteilung: **13.02.2013**
(21) Anmeldenummer: 07866211.1
(22) Anmeldetag: 10.12.2007
(51) Int. Cl.: C12N 9/88, C12N 5/10, C12P 13/00

(54) **(R)-HYDROXYNITRIL-LYASE AUS BRASSICACEEN**
(R)-HYDROXYNITRILE LYASE FROM BRASSICACEAE
(R)-HYDROXYNITRILE LYASE DE LA FAMILLE DES BRASSICACÉES

(30) Priorität: 08.12.2006 DE 102006058373
(43) Veröffentlichungstag der Anmeldung: 26.08.2009
(73) Patentinhaber: Evocatal Gmbh, 40225 Düsseldorf (DE)
(72) Erfinder: EGGERT, Thorsten, 45259 Essen (DE); ANDEXER, Jennifer, Cambridge CB2 7EQ (GB)
(74) Vertreter: Remus, Alvaro Johannes
(86) Internationale Anmeldenummer: PCT/DE2007/002216
(87) Internationale Veröffentlichungsnummer: WO 2008/067807

(56) Entgegenhaltungen:
- WO-A-03/016551
- WO-A-2004/083424
- WO-A-2004/090124
- DE-A1- 10 251 547
- DE-A1- 10 255 597
- DATABASE EMBL [Online] 24. September 2002 (2002-09-24), "Arabidopsis thaliana putative alpha-hydroxynitrile lyase (At5g10300) mRNA, complete cds." XP002480441 gefunden im EBI accession no. EMBL:AY142490 Database accession no. AY142490
- WEIS ROLAND ET AL: "Carving the active site of almond R-HNL for increased enantioselectivity" ANGEWANDTE CHEMIE. INTERNATIONAL EDITION, WILEY VCH VERLAG, WEINHEIM, Bd. 44, Nr. 30, 25. Juli 2005 (2005-07-25), Seiten 4700-4704, XP002382496 ISSN: 1433-7851
- FECHTER MARTIN H ET AL: "Hydroxynitrile lyases: Biological sources and application as biocatalysts" FOOD TECHNOLOGY AND BIOTECHNOLOGY, Bd. 42, Nr. 4, Oktober 2004 (2004-10), Seiten 287-294, XP002480439 ISSN: 1330-9862
- ANDEXER JENNIFER ET AL: "An R-selective hydroxynitrile lyase from Arabidopsis thaliana with an alpha/beta-hydrolase fold." ANGEWANDTE CHEMIE (INTERNATIONAL ED. IN ENGLISH) 2007, Bd. 46, Nr. 45, 19. November 2007 (2007-11-19), Seiten 8679-8681, XP002480440 ISSN: 1521-3773

### SEQUENCE LISTING

<110> Evocatal GmbH
<120> (R)-Hydroxynitril-Lyase aus Brassicaceen
<130> E05 168 PCT
<150> DE 10 2006 058 373.6
   <151> 2006-12-08
<160> 4
<170> PatentIn version 3.3
<210> 1
   <211> 258
   <212> PRT
   <213> Arabidopsis thaliana
<400> 1
   Met Glu Arg Lys His His Phe Val Leu Val His Asn Ala Tyr His Gly
   1               5                   10                  15
   Ala Trp Ile Trp Tyr Lys Leu Lys Pro Leu Leu Glu Ser Ala Gly His
               20                  25                  30
   Arg Val Thr Ala Val Glu Leu Ala Ala Ser Gly Ile Asp Pro Arg Pro
           35                  40                  45
   Ile Gln Ala Val Glu Thr Val Asp Glu Tyr Ser Lys Pro Leu Ile Glu
       50                  55                  60
   Thr Leu Lys Ser Leu Pro Glu Asn Glu Glu Val Ile Leu Val Gly Phe
   65                  70                  75                  80
   Ser Phe Gly Gly Ile Asn Ile Ala Leu Ala Ala Asp Ile Phe Pro Ala
                   85                  90                  95
   Lys Ile Lys Val Leu Val Phe Leu Asn Ala Phe Leu Pro Asp Thr Thr
               100                 105                 110
   His Val Pro Ser His Val Leu Asp Lys Tyr Met Glu Met Pro Gly Gly
           115                 120                 125
   Leu Gly Asp Cys Glu Phe Ser Ser His Glu Thr Arg Asn Gly Thr Met
       130                 135                 140
   Ser Leu Leu Lys Met Gly Pro Lys Phe Met Lys Ala Arg Leu Tyr Gln
   145                 150                 155                 160
   Asn Cys Pro Ile Glu Asp Tyr Glu Leu Ala Lys Met Leu His Arg Gln
                   165                 170                 175
   Gly Ser Phe Phe Thr Glu Asp Leu Ser Lys Lys Glu Lys Phe Ser Glu
               180                 185                 190
   Glu Gly Tyr Gly Ser Val Gln Arg Val Tyr Val Met Ser Ser Glu Asp
           195                 200                 205
   Lys Ala Ile Pro Cys Asp Phe Ile Arg Trp Met Ile Asp Asn Phe Asn
       210                 215                 220
   Val Ser Lys Val Tyr Glu Ile Asp Gly Gly Asp His Met Val Met Leu
   225                 230                 235                 240
   Ser Lys Pro Gln Lys Leu Phe Asp Ser Leu Ser Ala Ile Ala Thr Asp
                   245                 250                 255
   Tyr Met
<210> 2
   <211> 808
   <212> DNA
   <213> Arabidopsis thaliana
<400> 2
   atggagagga aacatcactt cgtgttagtt cacaacgctt atcatggagc ctggatctgg 60
   tacaagctca agcccctcct tgaatcagcc ggccaccgcg ttactgctgt cgaactcgcc 120
   gcctccggga tcgacccacg accaatccag gccgttgaaa ccgtcgacga atactccaaa 180
   ccgttgatcg aaaccctcaa atctcttcca gagaacgaag aggtaattct ggttggattc 240
   agcttcggag gcatcaacat cgctctcgcc gccgacatat ttccggcgaa gattaaggtt 300
   cttgtgttcc tcaacgcctt cttgcccgac acaacccacg tgccttctca cgttctggac 360
   aagtatatgg agatgcctgg aggtttggga gattgtgagt tttcatctca tgaaacaaga 420
   aatgggacga tgagtttatt gaagatggga ccaaaattca tgaaggcacg tctttaccaa 480
   aattgtccca tagaggatta cgagctggca aaaatgttgc ataggcaagg gtcatttttc 540
   acagaggatc tatcaaagaa agaaaagttt agcgaggaag gatatggttc ggtgcaacga 600
   gtttacgtaa tgagtagtga agacaaagcc atcccctgcg atttcattcg ttggatgatt 660
   gataatttca acgtctcgaa agtctacgag atcgatggcg gagatcacat ggtgatgctc 720
   tccaaacccc aaaaactctt tgactctctc tctgctattg ccaccgatta tatgtaataa 780
   tcttaagtcc gttttacttt tttctcat 808
<210> 3
   <211> 40
   <212> DNA
   <213> Artificial Sequence
<220>
   <223> 5' primer
<400> 3
   tataccatgg agaggaaaca tcacttcgtg ttagttcaca 40
<210> 4
   <211> 40
   <212> DNA
   <213> Artificial Sequence
<220>
   <223> 3' primer
<400> 4
   tatactcgag ttacatataa tcggtggcaa tagcagagag 40

## Beschreibung

Die Erfindung betrifft ein aus Pflanzen der Familie *Brassicaceae,* insbesondere der Gattung *Arabidopsis,* bevorzugt der Art *Arabidopsis thaliana,* isoliertes Polypeptid, mit der Aminosäuresequenz gemäß SEQ ID NR. 1, das zumindest die Aktivität einer Hydroxynitril - Lyase aufweist und (R) - selektiv ist. Die Erfindung betrifft ferner Verwendungen dieses Polypeptids und Nukleinsäuren, die für das erfindungsgemäße Polypeptid kodieren. Die Erfindung betrifft auch die Verwendung eines Polypeptids mit der Aminosäuresequenz gemäß SEQ ID NR. 1 und Verfahren, bei denen die Polypeptide als Katalysator eingesetzt werden.

### Hintergrund der Erfindung

Hydroxynitril-Lyasen (HNLs) (EC 4.1.2.10, EC 4.1.2.11, EC 4.1.2.37, EC 4.1.2.39) katalysieren die enantioselektive Spaltung von Cyanohydrinen zu einer Carbonylverbindung (Aldehyd oder Keton) und Blausäure.

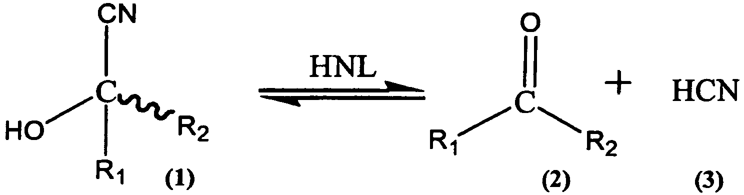

**HNL-katalysierte Reaktion:** In der natürlichen Reaktion wird ein Cyanohydrin **(1)** in eine Carbonylkomponente (Aldehyd oder Keton) **(2)** und Blausäure **(3)** gespalten, in der industriellen Anwendung wird aus den beiden letzteren ein chirales Cyanohydrin gebildet.

HNL-Aktivität wurde erstmals 1908 von Rosenthaler in Emulsin aus Mandeln detektiert. Vertreter dieser Enzymklasse finden sich überwiegend in Pflanzen, selten auch in Insekten und Bakterien. Alle bekannten HNLs wurden aus Pflanzen isoliert, die katalysierte Reaktion dient in der Natur der Abwehr von Fressfeinden. Diese Freisetzung von Blausäure aus Cyanohydrinen wird auch Cyanogenese genannt. Industriell genutzt wird die ebenfalls durch HNLs katalysierte Rückreaktion, d. h. die Bildung eines chiralen Cyanohydrins aus einem Aldehyd oder einem Keton und Blausäure. Chirale Cyanohydrine stellen wichtige Vorstufen zur Herstellung von z.B. β-Aminoalkoholen und α-Hydroxysäuren dar.

Die an sich sehr heterogene Enzymklasse kann grob in zwei Gruppen eingeteilt werden, die sich bezüglich des Vorkommens des Cofaktors FAD unterscheiden. Das essentielle FAD-Molekül scheint aber ausschließlich strukturelle Funktion zu haben, eine biochemische Bedeutung für die katalysierte Reaktion ist bislang nicht bekannt. FAD-haltige HNLs wurden bisher nur aus der Familie der *Rosaceen* beschrieben, ausführlich biochemisch charakterisierte Vertreter sind z. B. die Enzyme aus *Prunus amygdalus* (PaHNL, Mandel) und *Prunus mume* (PmHNL, Japanische Aprikose). Die Enzyme sind bezüglich ihrer Sequenz sehr ähnlich und weisen alle (*R*)-Selektivität bei der Bildung chiraler Cyanohydrine auf.

Die Gruppe der nicht-cofaktorhaltigen HNLs ist weitaus heterogener. Als Vertreter sind die Enzyme aus *Linum usitatissimum* (LuHNL, Leinsamen, (*R*)-selektiv) und *Sorghum bicolor* (SbHNL, Hirse, (*S*)-selektiv) zu nennen. Weiterhin sind in der Gruppe Enzyme aus *Hevea brasiliensis* (HbHNL, Parakautschukbaum) und *Manihot esculenta* (MeHNL, Maniok) vertreten. Während die HNL aus Leinsamen Ähnlichkeit zu zinkabhängigen Alkoholdehydrogenasen und die aus Hirse zu Carboxypeptidasen aufweist, gehören die zuletzt genannten Enzyme (wie auch Carboxypeptidasen) zur Gruppe der α/β-Hydrolasen und sind ausschließlich (*S*)-selektiv. Neben dem gemeinsamen Faltungsmotiv (s.u.) sind sich die beiden Proteine auch bezüglich ihrer Sequenz sehr ähnlich (77% Identität auf Aminosäureebene).

Neben den erwähnten HNLs aus Maniok und dem Parakautschukbaum gehören verschiedene Enzymgruppen wie Lipasen, Esterasen, Proteasen, Epoxidhydrolasen und Dehalogenasen zur Familie der α/β-Hydrolasen. Allen gemein ist das Faltungsmotiv bestehend aus meist parallelen β-Faltblattsträngen, die von α-Helices umgeben sind. Das aktive Zentrum wird von drei Resten, der so genannten katalytischen Triade gebildet (Ollis et al., 1992). Die Strukturen der beiden HNLs mit α/β-Hydrolase-Faltungsmotiv (HbHNL, MeHNL) sind aufgelöst und die katalytischen Reste eindeutig identifiziert.

Weitere Unterschiede zwischen den verschiedenen HNLs findet man hinsichtlich ihrer Substratspektren und Enantioselektivitäten. Überblick über die bekannten Enzyme geben die Artikel von Fechter et al. (2004) und Sharma et al. (2005).

Enzyme, die in industriellem Maßstab Anwendung finden, sollten ein breites Substratspektrum und hohe Enantioselektivität aufweisen. Weiterhin ist es vorteilhaft, wenn das entsprechende Enzym rekombinant hergestellt werden kann. Eine Zusammenfassung der wichtigsten HNLs und ihrer Eigenschaften ist in Tabelle 1 dargestellt.

**Tabelle 1: Übersicht über die Eigenschaften der industriell wichtigsten HNLs.**

| Ursprungsorganismus | Stereoselektivität | rekombinanter Expressionswirt | Substrate | | | | | Sonstiges | Literatur |
|---|---|---|---|---|---|---|---|---|---|
| | | | Aldehyde | | Ketone | | | | |
| | | | aliphatisch | aromatisch | aliphatisch | aromatisch | | | |
| *Manihot esculenta* bzw. MeHNL-"Tunnelmutante" W128A | S | *E. coli* | + | + | + | + | | | Bühler et al., 2003 |
| | | | | | Methylketone | | | | |
| *Hevea brasiliensis* | S | *E. coli* | + | + | + | | + | | Hasslacher et al., 1997 US 6337196 B1 |
| | | *P. pastoris* | | | Methylketone | | | | |
| | | S. *cerevisiae* | | | | | | | |
| *Prunus amygdalus* (Isoenzym 5) | R | *P. pastoris* | + | + | + | | + | Cofaktor FAD glykosiliert | Glieder et al., 2003 EP 1223220 B1 |
| *Linum usitatissimum* | R | *E. coli* | + | - | + | | - | | Albrecht et al., 1993 |
| | | *P. pastoris* | | | | | | | |

Die Produktion von chiralen Cyanohydrinen mit Hilfe von HNLs kann sowohl in wässrigen Systemen als auch in organischen Lösungsmitteln wie Diisopropylether (DIPE) durchgeführt werden. Häufig werden auch Zwei-Phasensysteme wie DIPE/Puffer angewandt. Der Vorteil in der Verwendung organischer Lösungsmittel liegt neben der guten Löslichkeit von Substraten und Produkten in der Unterdrückung der unkatalysierten chemischen Reaktion von Aldehyd/Keton und Blausäure zum racemischen Cyanohydrin. Da diese unerwünschte Reaktion temperaturabhängig ist und nur bei pH-Werten über pH 5 stattfindet, kann sie ebenfalls durch ein Absenken des pHs auf Werte unter 5 und eine relativ niedrige Reaktionstemperatur (<10°C) vermieden werden. In jüngster Zeit wurde zudem über erste Versuche zur Anwendung von HNLs in ionischen Liquiden berichtet (Gaisberger et al., 2004). Die jeweiligen HNLs werden in unterschiedlichen Präparationen eingesetzt; als gelöstes Enzym, Lyophilisat, Immobilisat auf diversen Trägermaterialien, CLECs (Cross-linked enzyme crystals) oder CLEAs (Cross-linked enzyme aggregates).

Aus der US 6 337 196 B1 ist beispielsweise die Herstellung von (S)-Cyanohydrinen mit der HNL aus *Hevea brasiliensis* bekannt. In der DE 100 62 306 A1 ist die Herstellung von (R)-Cyanohydrinen mit HNL aus *Prunus amygdalus* beschrieben. Weitere konkrete Beispiele für den Einsatz von (*R* )- und (*S*)-Cyanohydrinen finden sich in den Artikeln von Schmidt et al. (1999) und Fechter et al. (2004).

(*S*)-selektive Enzyme, die zurzeit industriell eingesetzt werden, sind die HNLs aus *Hevea brasiliensis* (HbHNL) und *Manihot esculenta* (MeHNL), beide können problemlos in mikrobiellen Wirten produziert werden und decken ein breites Substratspektrum (aliphatische und aromatische Aldehyde und Ketone, vorzugsweise Methylketone) ab. Das ebenfalls (*S*)-selektive Enyzme aus *Sorghum bicolor* spielt bislang so gut wie keine Rolle, da es nicht in heterologen Wirten produziert werden kann.

Aus der WO 2004/083424 A sind Expressionsvektoren bekannt, die Gene enthalten, die für mutierte (*R*)-Hydroxynitril-Lyasen aus Pflanzen der Familie Rosaceae kodieren. Diese mutierten HNLs weisen eine veränderte Substratspezifität auf und können zur Herstellung von enantiomerenreinen (*R*)- oder (*S*)-Cyanohydrinen verwendet werden.

In der EP 1 223 220 A1 wird die Verwendung der (*R*)-selektiven HNL aus *Prunus amygdalus* (PaHNL, Isoenzym 5) beschrieben. Das Enzym kann heterolog hergestellt werden, allerdings ist bislang nur eine Expression in einem eukaryotischen Wirt *(Pichia pastoris)* erfolgreich gewesen. Die ebenfalls (*R*)-selektive HNL aus Leinsamen (LuHNL) kann zwar heterolog in Bakterien exprimiert werden, hat aber die Einschränkung, dass ausschließlich aliphatische Substrate akzeptiert werden, die industrielle Anwendung ist dementsprechend begrenzt.

Deshalb besteht ein großes Interesse an der Auffindung weiterer (*R*)-selektiver HNLs mit neuen enzymatischen Eigenschaften, die insbesondere zur Umsetzung von aromatischen und aliphatischen Aldehyden und Ketonen geeignet sind und zudem mit guten Ausbeuten heterolog in bakteriellen Wirten exprimiert werden können.

### Kurzbeschreibung der Erfindung

Aufgabe der vorliegenden Erfindung ist es daher, neue HNLs bereitzustellen, die leicht zugänglich sind und die in der organischen Synthese zur Synthese chiraler (*R*)-Cyanohydrine verwendet werden können.

Die Aufgabe wird erfindungsgemäß durch ein Polypeptid mit der Aminosäuresequenz gemäß SEQ ID NR. 1 gelöst, das aus Pflanzen der Familie Brassicaceae, insbesondere der Gattung *Arabidopsis,* bevorzugt der Art *Arabidopsis thaliana,* isoliert werden kann, zumindest die Aktivität einer Hydroxynitril-Lyase aufweist, (*R*) - selektiv ist und durch einen oder mehrere der folgenden Aminosäure-Austausch(e) in Bezug auf SEQ ID NR. 1 gekennzeichnet ist:
a) Asparagin an Position 12 gegen Threonin (N12T) oder Alanin (N12A);
b) Tyrosin an Position 14 gegen Cystein (Y14C) oder Alanin (Y14A);
c) Leucin an Position 129 gegen Tryptophan (L129W),
wobei die Austausche Y14C und L129W eine Umkehr der Enantioselektivität bewirken..

Die erfindungsgemäße Hydroxynitril-Lyase ist die erste HNL aus der Familie der Brassicaceen. Die Pflanze, aus der dieses Enzym bzw. Gen isoliert wird, ist zudem als nicht cyanogen beschrieben. Alle bislang beschriebenen HNL-enthaltenden Pflanzen sind cyanogene Pflanzen, so dass bislang angenommen wurde, dass nur cyanogene Pflanzen Hydroxynitril-Lyasen enthalten. In *Arabidopsis* wurden bislang weder cyanogene Glykoside (stabile Lagerungsform der Cyanohydrine) noch Cyanohydrine nachgewiesen. Ein Vorkommen der katabolischen Enzyme wurde daher explizit ausgeschlossen (Wäspi et al., 1998). Obwohl die natürliche Funktion des Enzyms in *Arabidopsis* bisher nicht bekannt ist, ist das neue Enzym eine sehr interessante Alternative zu den bisher für die enzymatische Herstellung genutzten HNLs. Überraschend stellte sich nämlich heraus, dass das erfindungsgemäße Polypeptid (R)-selektiv ist. Aufgrund der Homologie zu den Enzymen aus Maniok und dem Parakautschukbaum, die beide eine charakteristische α/β-Hydrotasefaltung aufweisen, lag eine (S)-Selektivität nahe, da bisher alle Vertreter der HNLs, die besagtes Faltungsmotiv aufweisen, (S)-selektiv sind.

Eine besondere der Erfindung zugrunde liegende Aufgabe lag auch in der Eignung der HNL für die Akzeptanz von Carbonyl-Verbindungen mit aromatischen Seitenketten. Als solches sterisch anspruchsvolles Substrat sei beispielsweise Benzaldehyd genannt, das zu Mandelonitril führt. Das breite Substratspektrum des erfindungsgemäßen Polypeptids, welches sowohl aliphatische als auch aromatische Verbindungen umfasst, und die für die industrielle Anwendung adäquate Prozessstabilität ermöglichen vielfältige Anwendungsmöglichkeiten. Ein großer Vorteil ist weiterhin die Möglichkeit, das erfindungsgemäße Polypeptid mit wenig Aufwand und Kosten in *E. coli* zu produzieren.

Erfindungsgemäß basiert das erfindungsgemäße Polypeptid auf der Aminosäuresequenz gemäß SEQ ID NR. 1. Es konnte überraschend gezeigt werden, dass das erfindungsgemäß exprimierbare Polypeptid aus *Arabidopsis thaliana* (AtHNL) eine enzymatische Hydroxynitril-Lyase-Aktivität besitzen, obwohl *A. thaliana* nicht zu den cyanogenen Pflanzen gehört (Wäspi et al., 1998). Insbesondere die AtHNL entsprechend SEQ ID NR. 1 setzt Carbonyl-Verbindungen mit aromatischer oder aliphatischer Seitenkette um. Weiterhin zeichnet sich die erfindungsgemäße Hydroxynitril-Lyase durch ihre ausgezeichnete Stereoselektivität aus.

So werden selbst sterisch anspruchsvolle Substrate mit hohen Enantioselektivitäten von >95% ee in die gewünschten, korrespondierenden optisch aktiven Cyanohydrine umgesetzt.

Das erfindungsgemäße Polypeptid basiert also auf der Hydroxynitril-Lyase (AtHNL) aus *Arabidopsis thaliana* mit der folgenden Aminosäuresequenz (SEQ ID NR. 1) wobei sich das erfindungsgemäße Polypeptid von dem Polypeptid gemäß SEQ ID NR. 1 durch einen oder mehrere Aminosäure-Austausch(e) unterscheidet: Austausch des Asparagin an Position 12 gegen Threonin (N12T) oder Alanin (N12A), vorzugsweise in Kombination mit einem Austausch des Tyrosin an Position 14 gegen Cystein (Y14C) oder Alanin (Y14A). Durch diesen Austausch der Aminosäure an Position 12, ggf. in Kombination mit dem Austausch an Position 14, kann das Substratspektrum des erfindungsgemäßen Polypeptids in vorteilhafter Weise auf eine bessere Akzeptanz von Ketonen ausgedehnt werden. Ferner kann durch einen Austausch des Leucin an Position 129 gegen Tryptophan (L129W) und/oder den Austausch des Tyrosin an Position 14 gegen Cystein (Y14C) eine Umkehr der Enantioselektivität, d. h. von (*R*)-Selektivität zu (*S*)-Selektivität, erreicht werden.

Die erfindungsgemäßen Hydroxynitril-Lyasen können posttranslationale Modifikationen, wie z. B. Glycosylierungen oder Phosphorylierungen, aufweisen.

Die Erfindung umfasst ferner Proteine, insbesondere Fusionsproteine, die mindestens ein erfindungsgemäßes Polypeptid umfassen.

Ein weiterer Gegenstand der vorliegenden Erfindung sind Nukleinsäuren, codierend für die erfindungsgemäßen Polypeptide oder Proteine bzw. Hydroxynitril-Lyasen, oder DNA-Fragmente, die zu solchen, mit codierenden Nukleinsäuren unter stringenten Bedingungen hybridisierenden, Nukleinsäuresequenzen, komplementär sind.

Erfindungsgemäß sind diese Nukleinsäuren isolierte und/oder rekombinante Nukleinsäuremoleküle, die mindestens eine Nukleotidsequenz zur Synthese mindestens eines Polypeptids umfassen, wobei die Nukleotidsequenz ausgewählt ist aus der Gruppe bestehend aus:
a) Nukleotidsequenz, welche für ein erfindungsgemäßes Polypeptid kodiert;
b) Nukleotidsequenz, welche sich von der Nukleotidsequenz gemäß a) durch den Austausch zumindest eines Codons gegen ein synonymes Codon unterscheidet;
c) Nukleotidsequenz, welche dem komplementären Strang der Nukleotidsequenz gemäß a) entspricht.

Beispielsweise enthält das Hydroxynitril-Lyase-Gen *aus Arabidopsis thaliana* folgende codierende Nukleinsäuresequenz (SEQ ID NR. 2):

Die Information aus SEQ ID NR. 2 kann zur Erzeugung von Primern genutzt werden, um direkt homologe Formen mittels PCR zu identifizieren und zu klonieren. Darüber hinaus können aufgrund der Sequenzinformation Sonden zum Auffinden weiterer natürlich vorkommender funktioneller Varianten des *athnl*-Gens und damit der entsprechenden codierten Enzymvarianten benutzt werden. Ausgehend von SEQ ID NR. 2 oder von dazu allelen oder natürlich vorkommenden funktionellen Varianten kann z. B. über PCR unter Verwendung einer fehlerhaft arbeitenden DNA-Polymerase eine Bank künstlich erzeugter funktioneller Enzymvarianten erhalten werden. Mittels Standardmethoden werden einzelne Punktmutationen, die zu Aminosäureaustauschen führen, in die DNA-Sequenz eingebracht, durch die das Protein hinsichtlich seiner Eigenschaften wie. z. B. Substratspezifität verändert wird.

Erfindungsgemäß werden die folgenden Punktmutationen, einzeln oder in beliebiger Kombination, in die Nukleotidsequenz gemäß SEQ ID NR. 2 eingebracht:
a) Austausch des 2. Nukleotids (A = Adenin) des 12. Codons (AAC) gegen Cytosin (C), d.
   h. Umwandlung in das Codon mit der Nukleotidfolge ACC, oder alternativ Austausch des gesamten 12. Codons gegen das Codon ACT, ACA oder ACG bzw. GCT, GCC, GCA oder GCG;
b) Austausch des 2. Nukleotids (A = Adenin) des 14. Codons (TAT) gegen Guanin (G), d. h. Umwandlung in das Codon mit der Nukleotidfolge TGT, oder alternativ Austausch des gesamten 14. Codons gegen das Codon TGC bzw. GCT, GCC, GCA oder GCG;
c) Austausch des 2. Nukleotids (T = Thymin) des 129. Codons (TTG) gegen Guanin (G), d.
   h. Umwandlung in das Codon mit der Nukleotidfolge TGG;

Die codierenden DNA-Sequenzen können in herkömmliche Vektoren kloniert und nach Transformation von Wirtszellen mit solchen Vektoren in Zellkultur exprimiert werden. Geeignete Expressionsvektoren sind z. B. pET-28a(+) für *E*. *coli,* aber auch Expressionsvektoren anderer prokaryontischer Einzeller können verwendet werden. Als geeignete Expressionsvektoren für Hefen haben sich z. B. der pREP-Vektor oder der pINT-Vektor erwiesen. Für die Expression in Insektenzellen sind z. B. Baculovirus-Vektoren wie in EP-B1-0127839 oder EP-B1-0549721 offenbart, und für die Expression in Säugerzellen sind z. B. SV40-Vektoren geeignet, welche allgemein erhältlich sind.

Die Vektoren können neben den üblichen Markem, wie z. B. der Kanamycin-Resistenz, weitere funktionelle Nukleotidsequenzen zur Regulation, insbesondere zur Repression oder Induktion der Expression des HNL-Gens und/oder des Reportergens enthalten. Als Promotoren werden bevorzugt induzierbare Promotoren, wie z. B. der rha-Promotor oder der nmt1 - Promotor, oder starke Promotoren, wie z. B. der lac-, ara-, lambda-, pL-, T7- oder T3-Promotor, benutzt. Die codierenden DNA-Fragmente müssen in den Vektoren von einem Promotor aus transkribierbar sein. Weitere bewährte Promotoren sind z. B. der Baculovirus-Polyhedrin-Promotor für die Expression in Insektenzellen (s. z.B. EP-B1-0127839) oder der frühe SV40-Promotor oder die LTR-Promotoren z. B. von MMTV (Mouse Mammary Tumour Virus, Lee et al., 1981).

Die erfindungsgemäßen Expressionsvektoren können weitere funktionale Sequenzbereiche, wie z. B. einen Replikationsstartpunkt, Operatoren, Terminationssignale, Tags, die die Aufreinigung erleichtern (z.B. His-Tag, Strep-Tag) oder weitere Peptidsequenzen, durch die Fusionsproteine erzeugt werden, enthalten.

Mit den beschriebenen Vektoren können Wirtszellen mit den herkömmlichen Methoden, wie z. B. der Hitzeschock-Methode oder durch Elektroporation, transformiert werden.

Dadurch wird ein weiterer Gegenstand der vorliegenden Erfindung erhalten, dies sind Expressionssysteme, die Wirtszellen oder Wirtszellkulturen umfassen, die mit den eben beschriebenen Vektorsystemen transformiert sind. Bevorzugte Wirte sind einzellige prokaryontische Organismen, insbesondere *E*. *coli.* Zur Expression eukaryontischer erfindungsgemä-βer *hnl*-Gene kann es von Vorteil sein, eukaryontische Expressionssysteme heranzuziehen, um z. B. eukaryontentypische posttranslationale Modifikationen in das *hnl*-Genprodukt einzuführen. Besonders geeignete eukaryontische Wirtszellen sind Hefen.

Ein bevorzugtes Expressionssystem enthält ein Hydroxynitril-Lyase-Gen gemäß SEQ ID NR. 2 mit einer oder mehreren der oben beschriebenen Mutationen in einem zur Expression in *E*. *coli* geeigneten Vektor, wie z. B. einem pET-28a(+)-Vektor, wobei das Hydroxynitril-Lyase-Gen transkribierbar in den Vektor kloniert sein muss. Bevorzugt wird das eingeschleust *hnl-*Gen so in den pET-28a(+)-Vektor kloniert, das die Transkription unter der Kontrolle des im Vektor vorhandenen IPTG-induzierbaren Promotors steht. Alternativ werden auch Rhamnose-induzierte Promotoren bevorzugt eingesetzt.

Die Expressionssysteme können unter Anwendung von dem Fachmann bekannten Standardprotokollen kultiviert werden. Die Expression des in das Expressionssystem geschleusten Gens kann je nach Transkriptionskontrolle und verwendeten Vektor entweder konstitutiv oder, wie z. B. im Falle der Benutzung von pET-28a(+) als Expressionsplasmid unter Zugabe von IPTG, reguliert werden. Nach Expression einer erfindungsgemäßen Hydroxynitril-Lyase erfolgt deren Aufreinigung z. B. mittels Chromatographie oder Zentrifugation. Zur Durchführung katalytischer Reaktionen können die so gereinigten Enzyme, aber auch die Rohextrakte bzw. Zentrifugationsüberstände oder -fraktionen direkt verwendet werden. Die Enzyme können sowohl als in wässrigem Puffer gelöster Katalysator, Lyophilisat oder Immobilisat auf verschiedenen Trägermaterialen (sowohl kovalente als auch nicht-.kovalente Bindung sowie als CLEAs oder CLECs) zum Einsatz kommen.

Die Erfindung umfasst also ferner Zellen, welche zumindest ein erfindungsgemäßes Nukleinsäuremolekül oder mindestens einen ein solches enthaltenden Vektor beinhalten.

Die Erfindung umfasst auch die Verwendung des erfindungsgemäßen Polypeptids, oder des erfindungsgemäßen Proteins, oder der erfindungsgemäßen Zelle, zur Herstellung von chiralen Cyanohydrinen. Somit ist ein weiterer Gegenstand der vorliegenden Erfindung die Verwendung von erfindungsgemäßen Hydroxynitril-Lyasen zur katalytischen Herstellung von chiralen Cyanohydrinen.

Die Erfindung umfasst ferner die Verwendung eines Polypeptids mit der Aminosäuresequenz gemäß SEQ ID NR. 1, das aus Pflanzen der Familie Brassicaceae, insbesondere der Gattung *Arabidopsis,* bevorzugt der Art *Arabidopsis thaliana,* isoliert wurde und das zumindest die Aktivität einer Hydroxynitril - Lyase aufweist, zur (*R*)-selektiven Herstellung von chiralen Cyanohydrinen.

Die Erfindung umfasst darüber hinaus Verfahren zur Synthese von chirafen Cyanohydrinen aus mindestens einer Carbonylverbindung und Blausäure, bei dem die Reaktion in Anwesenheit des erfindungsgemäßen Polypeptids, oder des erfindungsgemäßen Proteins, oder der erfindungsgemäßen Zelle, durchgeführt wird. Dabei kann als Carbonylverbindung ein aliphatischer oder aromatischer Aldehyd eingesetzt werden. Alternativ kann aber als Carbonylverbindung auch ein aliphatisches oder aromatisches Keton eingesetzt werden. Die Carbonyl-Verbindungen können auch mit anderen Nucleophilen anstelle von HCN umgesetzt werden.

Die Erfindung umfasst ferner ein Verfahren zur (R)-selektiven Synthese von chiralen Cyanohydrinen aus mindestens einer Carbonylverbindung und Blausäure, bei dem die Reaktion in Anwesenheit eines Polypeptids mit der Aminosäuresequenz gemäß SEQ ID NR. 1, das aus Pflanzen der Familie Brassicaceae, insbesondere der Gattung *Arabidopsis,* bevorzugt der Art *Arabidopsis thaliana,* isoliert wurde und das zumindest die Aktivität einer Hydroxynitril - Lyase aufweist, durchgeführt wird.

Die Erfindung umfasst auch ein Verfahren zur Spaltung von Cyanohydrinen in mindestens eine Carbonylverbindung und Blausäure, bei dem die Reaktion in Anwesenheit des erfindungsgemäßen Polypeptids, oder des erfindungsgemäßen Proteins, oder der erfindungsgemäßen Zelle, durchgeführt wird.

Die Erfindung umfasst ferner ein Verfahren zur (*R*)-selektiven Spaltung von Cyanohydrinen in mindestens eine Carbonylverbindung und Blausäure, bei dem die Reaktion in Anwesenheit eines Polypeptids mit der Aminosäuresequenz gemäß SEQ ID NR. 1, das aus Pflanzen der Familie Brassicaceae, insbesondere der Gattung *Arabidopsis,* bevorzugt der Art *Arabidopsis thaliana,* isoliert wurde und das zumindest die Aktivität einer Hydroxynitril - Lyase aufweist, durchgeführt wird.

### Kurze Beschreibung der Abbildungen

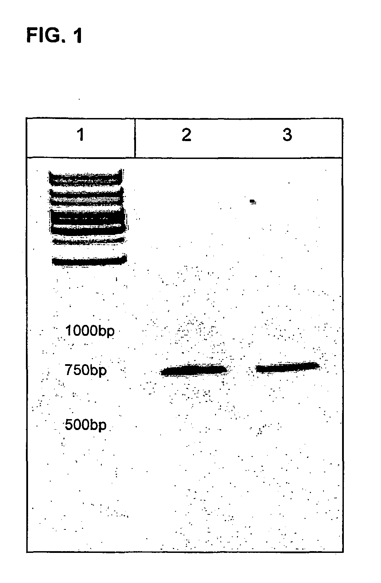

Die Erfindung wird im Folgenden anhand der Figuren und der folgenden Beispiele exemplarisch näher erläutert. Es zeigt
**Figur 1** ein DNA-Agarosegel mit PCR-Produkten (AtHNL) aus cDNA aus *Arabidopsis-*Keimlingen: 1 = Marker, 2 und 3 = PCR-Produkte (AtHNL, das PCR-Produkt hat eine Größe von etwa 780 Basenpaaren und entspricht somit der Länge des Gens in den Datenbanken);

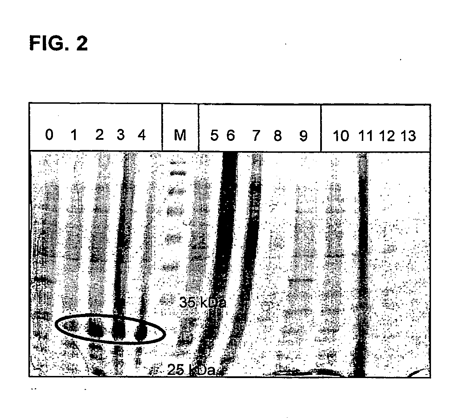

**Figur 2** ein SDS-Polyacrylamidgel zur Kontrolle der induzierten Gen-Expression in *E*. *coli -* Zellen, die das Plasmid pAtHNL enthalten: M = Marker, 0-4 = induziert, 5-9 = nicht induziert, 10-13 = Leervektor, zur Kontrolle der Expression wurden ab dem Zeitpunkt der Proteinexpression (Zusatz von IPTG) genommene Proben der Kultur auf ein SDS-Gel aufgetragen (0-4 h nach Induktion), als Kontrollen dienen eine nicht induzierte Kultur und der Leervektor pET28a, auf der erwarteten Höhe von ca. 30kDa ist in der induzierten Kultur eine mit der Zeit zunehmende Bande zu sehen (Markierung), in beiden Kontrollen nicht;

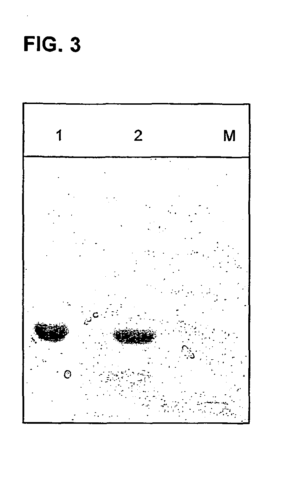

**Figur 3** ein SDS-Polyacrylamidgel zum Nachweis der Aufreinigung des erfindungsgemäßen Polypeptids (AtHNL): M = Marker, 1 = nach Q-Sepharose, 2 = nach Gelfiltration, aufgeschlossener Zellrohextrakt wird zunächst über einen Anionenaustauscher (Q-Sepharose) gereinigt, danach schließt sich ein zweiter Reinigungsschritt mittels Gelfiltrationschromatographie an;

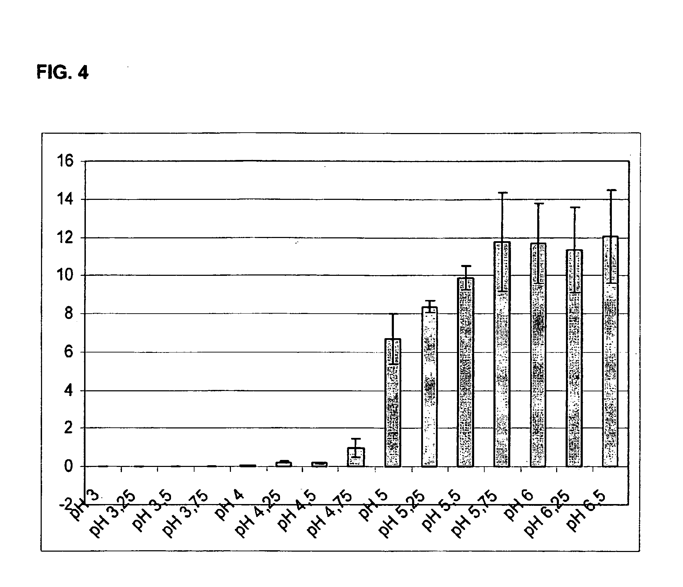

**Figur 4** ein Balkendiagramm der enzymatischen Aktivität des erfindungsgemäßen Polypeptids bei unterschiedlichen pH-Werten: Das pH-Optimum der AtHNL liegt etwa bei pH 5,75, Aktivität ist ab pH 4,25 zu messen, ab pH 5 steigt die Aktivität sprunghaft an;

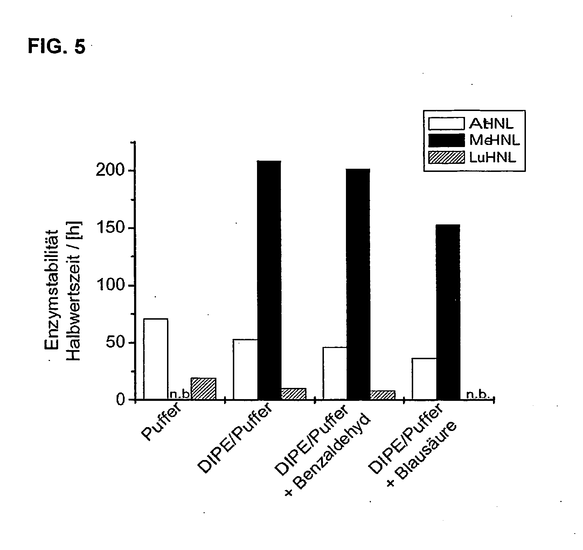

**Figur 5** ein Balkendiagramm der Stabilität des erfindungsgemäßen Polypeptids unter Reaktionsbedingungen im Vergleich mit den Hydroxynitril-Lyasen aus Maniok und Leinsamen: Im Vergleich mit der (*S*)-selektiven HNL aus Maniok und der (*R*)-selektiven HNL aus Leinsamen sind die Halbwertszeiten unten Reaktionsbedingungen für die AtHNL aus *Arabidiopsis* aufgetragen;

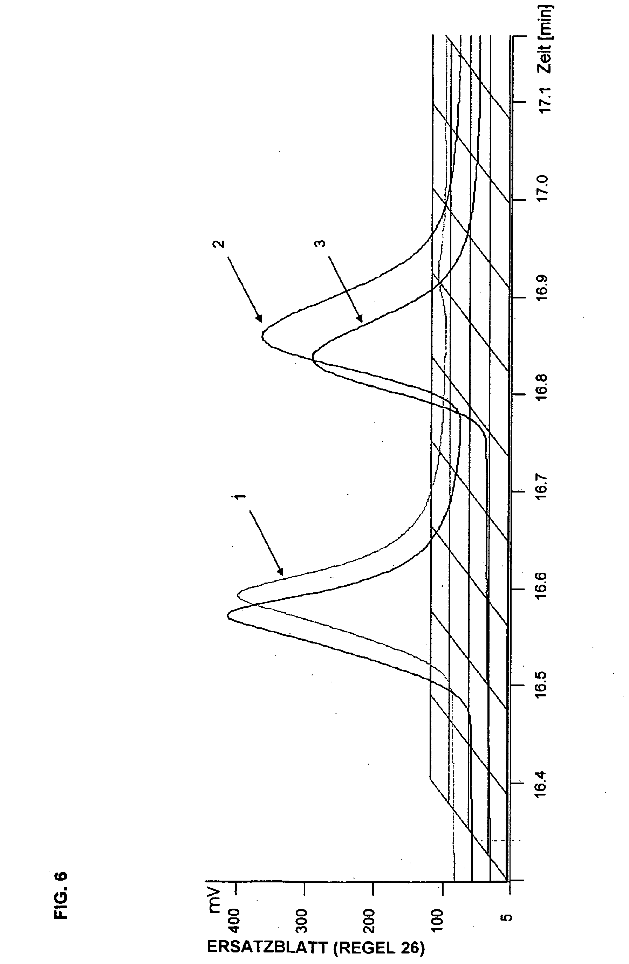

**Figur 6** ein Diagramm zum Nachweis der Stereoselektivität des erfindungsgemäßen Polypeptids mittels chiraler Gaschromatographie: 1 = Produkt der AtHNL: (R)-Mandelonitril, 2 = racemisches Mandelonitril, 3 = (S)-Mandelonitril, untersucht wurde das Produkt der AtHNL bei der Synthese von Mandelonitril aus Benzaldehyd und HCN, als Vergleich dienen reines (*S*)-Mandelonitril und racemisches Mandelonitril; und

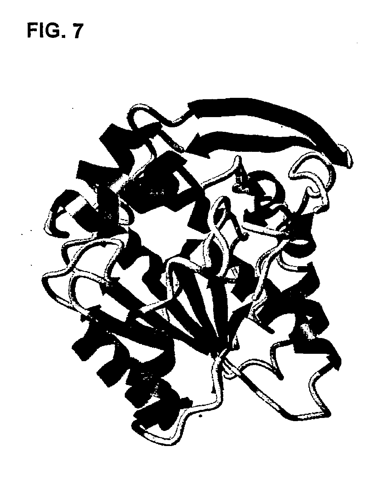

**Figur 7** ein Strukturmodell des erfindungsgemäßen Polypeptids auf Basis der Kristallstruktur der Hydroxynitril-Lyase aus *Hevea brasiliensis:* Das Modell wurde auf Basis der Kristallstruktur der homologen HbHNL erstellt.

### Beschreibung beispielhafter und bevorzugter Ausführungsformen der Erfindung

Im Folgenden wird beispielhaft ein erfindungsgemäßes Polypeptid aus der nicht-cyanogenen Pflanze *Arabidopsis thaliana* beschrieben. Die dargestellten Resultate werden durch detaillierte Beispiele verdeutlicht und belegt.

Bei einer mit der Aminosäuresequenz der MeHNL durchgeführten Datenbankrecherche stellte sich heraus, dass es in der Pflanze *Arabidopsis thaliana* eine Vielzahl homologer Proteine gibt, für die eine Hydroxynitril-Lyase-Aktivität aber nie beschrieben wurde. Die Pflanze *Arabidopsis thaliana* (Ackerschmalwand) ist ein Modellorganismus (übersichtliches Genom, kurze Generationszeit) und dementsprechend umfassend untersucht. Das Genom ist vollständig sequenziert und in Sequenzdatenbanken einzusehen. Beispiele für solche Datenbanken sind z.B. "Genbank" (http://www.ncbi.nlm.nih.gov/Genbank/index.html) als Sammlung aller veröffentlichten Sequenzen oder TAIR (www.arabidopsis.org) als eine auf einen Organismus (hier *Arabidopsis*) beschränkte Datenbank. Aufgrund der großen Fortschritte in der Molekularbiologie gehen zu viele neue Sequenzen in die entsprechenden Datenbanken ein, als dass sie alle einzeln beurteilt und beschrieben werden könnten. Daher werden bisher nicht weiter untersuchte Gene, die aber als echte Gene identifiziert worden sind, aufgrund ihrer Ähnlichkeiten zu bekannten Sequenzen automatisch durch entsprechende Computer-Algorithmen annotiert. Ob die in dieser Beschreibung genannte Funktion tatsächlich zutrifft, muss jedoch für jedes Protein einzeln bestimmt werden. Einige der zur HNL aus Maniok homologen, als putative HNLs annotierte Proteine aus *Arabidopsis* wurden im Zuge der Arbeiten bereits kloniert, heterolog in *E. coli* exprimiert und auf HNL-Aktivität (mit den Substraten Acetoncyanohydrin und Mandelonitril) getestet, eine HNL-Aktivität konnte allerdings bei vier der fünf bisher untersuchten Proteine nicht festgestellt werden. Überraschenderweise zeigt das fünfte Protein (At5g10300) hohe Aktivität bezüglich der Spaltung von Mandelonitril (s. Beispiel 6 (la)).

Die Gensequenz der neuen HNL (im Folgenden AtHNL) stammt wie oben beschrieben aus *Arabidopsis thaliana* (Ackerschmalwand) und weist große Homologie zu den Enzymen aus *Hevea brasiliensis* und *Manihot esculenta* auf. Das Gen (s. SEQ ID NR. 1) ist in der entsprechenden Literatur über α/β-Hydrolase verwandte HNLs als homologe Sequenz beschrieben, eine HNL-Aktivität wurde allerdings nicht vermutet, da *Arabidopsis* nicht als zur Cyanogenese befähigte Pflanze bekannt ist (Wäspi, 1998). In den einschlägigen Datenbanken taucht das Gen unter der Accession-Nummer NP_196592 (Genbank) bzw. At5g10300 (TAIR-Nomenklatur) auf und wird automatisch als putative Hydroxynitril-Lyase annotiert. In der Literatur ist dieses Gen nicht als HNL beschrieben. Die Bezeichnung findet sich neben den schon erwähnten HNL-Veröffentlichungen in Untersuchungen des *Arabidopsis*-Genoms oder bestimmter Teile (z. B. durch Wasserstress regulierte Gene (Bray, 2002)).

Nur eines von fünf getesteten Enzymen, die alle signifikante Ähnlichkeit zu den Enzymen aus Maniok und dem Parakautschukbaum aufweisen, hat tatsächlich die vorhergesagte Aktivität. Folglich lässt sich auch in diesem Fall alleine aufgrund des Sequenzvergleiches keine Aussage über die Aktivität eines Proteins treffen. Bezüglich der Sequenz interessante Gensequenzen müssen daher einzeln kloniert, exprimiert und auf Aktivität getestet werden.

### Beispiel 1: Datenbankrecherche

Mit der Aminosäuresequenz der HNL aus *Manihot esculenta* wird eine Datenbanksuche (Protein-Protein Blast, Standardeinstellungen, http://www.ncbi.nlm.nih.gov/blastl) durchgeführt, die Ergebnisse werden auf den Organismus *Arabidopsis thaliana* gefiltert. Die resultierende Liste enthält 22 Sequenzen mit mindestens 47 % Ähnlichkeit auf Aminosäuresequenzebene, von denen 5 Sequenzen in Tabelle 2 aufgeführt sind. Die in Tabelle 2 aufgeführten Gene werden kloniert, exprimiert und auf HNL-Aktivität untersucht.

**Tabelle 2: Liste mit MeHNL-ähnlichen Genen aus Arabidopsis thaliana**

| **TAIR-Nomenklatur** | **Accession Nr.** | **Ahnlichkeit zu MeHNL (%)** | **Anmerkung/Sonstiges** |
|---|---|---|---|
| At4g0990 | NM 117058 | 50 | kloniert und exprimiert, keine Aktivität |
| At3q10870 | AY096692 | 48 | kloniert und exprimiert, keine Aktivität |
| Ast3150440 | AY142031 | 57 | kloniert und exprimiert, keine Aktivität |
| At4q37150 | BT006227 | 56 | kloniert und exprimiert, keine Aktivität |
| At5g10300 | NP 196592 | 67 | kloniert und exprimiert, HNL-Aktivität (ein erfindungsgemäßes Polypeptid) |

### Beispiel 2: Amplifizierung des Gens At5g10300 (im Folgenden AtHNL) aus cDNA, Klonierung in den Expressionsvektor pET28a und Transformation in E.coli

Aus *Arabidopsis*-Keimlingen wird mit Hilfe des "RNeasy® Plant Mini Kits" von Qiagen die mRNA isoliert, die Prozedur wird nach der im Kit enthaltenen Anleitung durchgeführt. Mittels des "RevertAid™ First Strand cDNA Synthesis Kits" von Fermentas wird die erhaltene mRNA in cDNA umgeschrieben. Diese dient als Zielstruktur zur Amplifizierung der HNL-homologen Gene. Die PCR wird mit sequenzspezifischen Primern (SEQ ID NRN. 3 und 4) bei einer Annealingtemperatur von 58°C durchgeführt. Nach Aufreinigung des PCR-Produktes aus einem DNA-Agarosegel (**Figur 1**) mit einem handelsüblichen Gelelutionskit werden mit den Restriktionsendonucleasen NcoI und Xhol überlappende Enden erzeugt. Im Anschluss kann das Fragment in den ebenfalls mit den genannten Restriktionsenzymen geschnittenen Vektor pET28a kloniert werden. Die Ligation findet für 16 h bei 16°C statt. Die Sequenz des so klonierten Inserts wird durch Sequenzierung bestätigt.

Das oben beschriebene Plasmid (pAtHNL) kann in den bakteriellen Wirt *Escherichia coli* transformiert und das entsprechende Genprodukt exprimiert werden. Hierzu werden kompetente *E*. *coli* BL21(DE3) Zellen (chemisch oder elektrokompetent) nach Standardmethoden mit pAtHNL transformiert. Erfolgreich transformierte Klone können auf LB-Agarplatten mit dem Antibiotikum Kanamycin selektiert werden.

### Beispiel 3: Expression des Plasmids in E. coli

Zur Expression der HNL wird zunächst eine Übernachtkultur von *E. coli* (BL21 (DE3), transformiert mit pAtHNL) bei 30°C in LB-Medium mit 50µg/mL Kanamycin mit einer Einzelkolonie angeimpft. Am nächsten Morgen wird die Hauptkultur (ebenfalls LB-Medium/Kanamycin) im Verhältnis 1:20 mit der Vorkultur überimpft. Nachdem eine optische Dichte von 0,6 bis 0,8 bei 550 nm erreicht ist, werden die Kulturen mit 0,4 mM IPTG induziert. Nach 5 h Wachstum bei 30°C können die Zellen durch Zentrifugation geerntet und bei -20°C gelagert werden. Der Erfolg der Expression wird auf einem SDS-Polyacrylamidgel kontrolliert (**Figur 2**). Aus der Aminosäuresequenz ergibt sich ein theoretisches Molekulargewicht von 29,2 kDa für die Untereinheit der AtHNL.

### Beispiel 4: Aufreinigung des Proteins

Abzentrifugierte Zellen (15-20g, Beipiel 3) werden in Aufschlusspuffer (50mM KPi, pH 5,5) resuspendiert und auf eine äqulibrierte Q-Sepharose-Säule (Bettvolumen ca. 25mL) aufgetragen. AtHNL-aktive Fraktionen werden durch Aktivitätstests (Mandelonitrilspaltung, s. Beispiel 6, la) identifiziert.

Ein Teil des Proteins eluiert zum Ende des Durchlaufs, der Rest bei sehr geringen Salzkonzentrationen (ca.0.15 M NaCl). Über den Salzgradienten eluierte Fraktionen werden über eine Sephadex G-25 Säule (1 L Bettvolumen, Puffer: 10mM KPi, pH 6) entsalzt. Die eluierten Fraktionen der G-25 werden vereinigt und lyophilisiert. Zur weiteren Aufreinigung kann das Lyophilisat (ca. 250mg) in wenig Puffer (10mM Acetat pH 6, 150mM NaCl) aufgenommen und über eine Gelfiltrationssäule (Sephadex G-200, Bettvolumen 125mL) gegeben werden (**Figur 3**). Die aktiven Fraktionen werden im Anschluss entweder erneut lyophilisiert oder in Centricons (VivaSpin (VivaScience), Cutoffvolumen 10kDa) aufkonzentriert.

### Beispiel 5: Proteinchemische Charakterisierung

### a. molekulare Masse

Die berechnete molekulare Masse des Proteins von ca. 30 kDa kann durch SDS-Gelelektrophorese bestätigt werden (s. Figuren 2 und 3).

### b. oligomerer Zustand

Nach Kalibrierung der Sephadex G200-Säule mit Ribonuclease A (13,7kDa), Chymotrypsinogen A (25kDa), Ovalbumin (43kDa), Albumin (67kDa), Aldolase (158kDa), Catalase (232kDa) und Blue Dextran 2000 (2000 kDa) ergibt sich für das native Protein ein Molekulargewicht von ca. 50 kDa. Dies lässt auf ein Vorkommen des Proteins als Dimer schließen (Monomer berechnet aus Sequenz: 29,2kDa).

### c. pH Optimum

Das pH Optimum wurde mit dem in Beispiel 6 (II) beschriebenen Enzymtest ermittelt. Geringe Aktivität ist reproduzierbar bereits ab pH 4,25 zu messen, bei pH 5 steigt die Aktivität sprunghaft an. Bei pH 5,75 ist der Maximalwert erreicht, bis zum höchsten aufgenommenen Messwert (pH 6,5) bleibt dieser Wert gleich (**Figur 4**). Werte oberhalb pH 6,5 können nicht gemessen werde, da der durch den Eigenzerfall des Substrates verursachte Hintergrund zu groß wird.

### d. Stabilität unter Reaktionsbedingungen

Um die Eignung des Proteins für den Einsatz in industriellen Anwendungen abschätzen zu können, muss das Protein im Hinblick auf die Stabilität im dort verwendeten Reaktionssystem untersucht werden. Als Beispiel für ein häufig in großtechnischen Verfahren verwendetes Reaktionssystem dient ein Zweiphasensystem aus Puffer und Diisopropylether (1:1). Die Inkubationsbedingungen entsprechen den in Beispiel 6 (II) geschilderten Bedingungen aus der Synthesereaktion. Die verbleibende Aktivität nach verschiedenen Verweilzeiten wird mittels eines leicht modifizierten Spaltungstests (vgl. Beispiel 6 (Ia)) ermittelt. Als Vergleich dienen die (S)-selektive HNL aus *Manihot esculenta* (MeHNL) und die (*R*)-selektive HNL aus *Linum usitatissimum* LuHNL. Im direkten Vergleich zum homologen Protein MeHNL sind die für die AtHNL ermittelten Stabilitätszeiten deutlich kürzer, im Vergleich zum ebenfalls (*R*)-selektiven Enzym LuHNL sind die Werte allerdings deutlich besser (bis zu viermal höher, **Figur 5**)). Alle Halbwertszeiten liegen aber über den im Allgemeinen verwendeten Maximalzeiten für Cyanohydrinsynthesen (10-24 h).

### Beispiel 6: Substratspektrum und Stereoselektivität

Die HNL-Aktivität wird sowohl durch die Spaltung von Cyanohydrinen als auch durch die Synthese von Cyanohydrinen nachgewiesen. Hierzu werden verschiedene Aktivitätstests herangezogen. Zur besseren Übersicht werden alle verwendeten Testsysteme im Vorfeld beschrieben, die Resultate sind im Anschluss dargestellt.

### Ia) Spaltung von Mandelonitril (=Benzaldehydcyanohydrin), Nachweis des freigesetzten Benzaldehyds (Küvettentest):

Das verwendete Protokoll basiert auf dem 1999 von Bauer et al. beschriebenen Test und wurde nur leicht modifiziert. 700µl Citrat-Phosphatpuffer pH5 (48,5 mL 0,1M Zitronensäure, 51,5 mL 0,2M Dikaliumhydrogenphosphatad 100ml Wasser) werden mit 100µl Enzymprobe in einer Quarzglassküvette gemischt, mit 200µl Mandelonitrillösung (60mM in Citrat-Phosphatpuffer pH3,5 (1,82mL 0,1M Zitronensäure, 1,06mL 0,1M DiNatriumhydrogenphosphat ad 100mL Wasser) wird die Reaktion gestartet. Der Anstieg der Extinktion bei 280nm wird für 2 min verfolgt. Er ist proportional zur Benzaldehydkonzentration in der Probe. Der Eigenzerfall des Cyanohydrins wird in einer Kontrollprobe ohne Enzym erfasst und von den gemessenen Werten für die jeweilige Enzymprobe abgezogen. Zur Bestimmung der Restaktivität im Zuge der in Beispiel 5d beschriebenen Stabilitätsuntersuchungen wurde ein leicht abgeändertes Verfahren genutzt: 870µl Citratpuffer (50mM, pH4) werden mit 30pl der Enzymprobe versetzt, mit der Zugabe von 100 µl Mandelonitril (60mM in 50 mM Citratpuffer pH2) wird die Reaktion gestartet und über 2 Minuten bei 280nm verfolgt. Der Vorteil bei dieser Variante ist, dass die nicht-enzymatische Reaktion nahezu vollständig unterdrückt wird, nichtsdestotrotz wird die Kontrollreaktion ohne Enzym bei jeder Messreihe mit verfolgt. Die Enzymaktivität ist bei diesem Verfahren jedoch deutlich geringer.

### Ib) Spaltung von verschiedenen Cyanohydrinen, Nachweis der entstehenden Blausäure (Microtiterplattentest):

140µl Citrat-Phosphatpuffer pH5 (s. Ia) werden mit 10µl Enzymprobe versetzt. Wie auch bei Ia beschrieben, wird bei jeder Messreihe eine Kontrolle (Eigenzerfall des jeweiligen Cyanohydrins in Abwesenheit des Enzyms) betrachtet und von den ermittelten Werten abgezogen. Die enzymatische Reaktion wird mit 10µl Substratlösung (ein beliebiges Cyanohydrin, 300mM in 0,1 M Zitronensäure) gestartet und nach 5 min mit 10µl 100 mM N-Chlorosuccinimid (mit 10fachen Überschuss (w/w) Succinimid) abgestoppt. Die entstandene Blausäure reagiert mit N-Chlorosuccinimide und der im Anschluss zugegebenen Mischung aus Isonicotinsäure und Barbitursäure (65mM/125mM in 0,2 M Natronlauge) zu einem violetten Farbstoff, dessen Bildungsgeschwindigkeit mit der HCN-Menge in der Probe korreliert (Andexer et al., 2006).

### II. Synthese von Cyanohydrinen:

Als Beispiel für die Synthese wird hier eine Umsetzung im Zweiphasensystem verwendet. Andere Möglichkeiten wie die Synthese in rein organischem Lösungsmittel (auch z. B. mit immobilisiertem Enzym), in wässrigen Systemen sowie in anderen nicht-konventionellen Lösungsmitteln wie ionischen Liquiden sind ebenfalls die Synthese von Cyanohydrinen anwendbar. Als Reaktionssystem wird ein Zweiphasensystem aus Citratpuffer pH4 und Diisopropylether im Verhältnis 1:1 (gesamt 5 mL) verwendet. Die Reaktion mit 20 U/mL gereinigter, lyophilisierter HNL findet bei 10°C und 400rpm statt. Die Substrate werden im Verhältnis 1:5 (50mM Aldehyd/Keton und 250mM HCN) eingesetzt. Je nach Reaktionsgeschwindigkeit werden die Substrate für 1 h bis mehrere Tage umgesetzt. Die entnommenen Proben werden nach Derivatisierung (500µL Dichlormethan, 50µl Trifluoressigsäureanhydrid, 50µl Pyridin + 50µl Probe aus der organischen Phase) gaschromatographisch untersucht. Hierzu wird der Gaschromatograph CP-3800 der Firma Varian (FID-Detektor) mit einer CP Chirasil-DEX CB Säule (Länge 25m, Innendurchmesser 0,25mm, Filmdicke 0,25µm) derselben Firma verwendet. Als Trägergas wird Helium mit einer Flussgeschwindigkeit von 2mUmin eingesetzt. Folgendes Temperaturprogramm findet für nahezu alle Produkte bis auf 3-Pherioxybenzaldehydcyanohydrin Verwendung: Die Säule wird für 1 min bei 50°C gehalten, dann wird mit einer Heizrate von 3°/min auf 110°C aufgeheizt, diese Temperatur wird für 15 min gehalten. Für 3-Phenoxybenzaldehydcyanohydrin wird die Säule für 1 min bei 110°C gehalten, dann wird mit einer Heizrate von 5°/min auf 130°C aufgeheizt, diese Temperatur wird für 80 min gehalten.

Unerwartet ist die Tatsache, dass das Enzym (*R*)-selektiv ist, da die bisher bekannten HNLs aus der Familie der α/β-Hydrolasen alle das (*S*)-Enantiomer bevorzugen. Die umgesetzten Substrate umfassen sowohl aliphatische als auch aromatische Verbindungen, Aldehyde werden gegenüber Ketonen bevorzugt akzeptiert.

### a) Aktivitäten für Spaltung von Cyanohydrinen:

Für die Spaltung verschiedener kommerziell erhältlicher Cyanohydrine sind Aktivitäten gemessen worden, die Vergleichbarkeit der einzelnen Substrate untereinander ist allerdings nur sehr eingeschränkt möglich, da die Substrate a) zum Teil racemisch und zum anderen Teil achiral sind und b) die Reinheit der kommerziell erhältlichen Stoffe sehr stark variiert. Durch Verunreinigungen der Substrate mit dem korrespondierenden Aldehyd oder Keton kann das Enzym inhibiert werden, dieser Effekt tritt beispielsweise bei der HNL aus *Manihot esculenta* auf, die durch Benzaldehyd (Verunreinigung in Mandelonitril) inhibiert wird. Die Betrachtung der Spaltungsreaktion dient aus den genannten Gründen nur einem ersten Überblick (Tab. 3). Aussagekräftigere Ergebnisse werden durch die Untersuchung der Synthese von Cyanohydrinen mit anschließender gaschromatographischer Analyse erzielt.

**Tabelle 3: Substratspektrum der AtHNL (Spaltungsreaktion)**

| Substrat (Cyanohydrin) | | | spezifische Aktivität (U/mg) | |
|---|---|---|---|---|
| | R¹ | R² | HCN-Test (s.o., lb) | Benzaldehyd-Test (s.o., la) |
| Acetaldehydcyanohydrin | H | H | <0,05 | |
| Propionaldehydcyanohydrin | H | CH₃ | <0,05 | |
| Benzaldehydcyanohydrin | H | 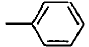 | 4,3 | 12,5 |
| 3-Phenoxybenzaldehydcyanohydrin | H | 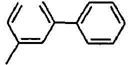 | 0,3 | |
| Acetoncyanhydrin | CH₃ | CH₃ | <0,05 | |
| Cyclohexanoncyanohydrin | 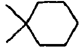 | | 0,11 | |

### b) Aktivitäten für die Synthese von Cyanohydrinen aus Aldehyden/Ketonen und Blausäure im Zweiphasensystem:

Für die AtHNL wurde ein partielles Substratspektrum aufgenommen (Tab. 4). Betrachtet wird die Syntheserichtung, der entsprechende Aldehyd wird in einem Zweiphasensystem mit Blausäure versetzt (Ergebnisse in Tab. 4). Alle getesteten Aldehyde werden umgesetzt, bei Ketonen lässt sich nur geringe Aktivität nachweisen:

### c) Nachweis der Stereoselektivität

Die Stereoselektivität wurde zum einen mittels chiraler Gaschromatographie bestimmt (**Figur 6**), zum anderen im Spaltungstest mit enantiomerenreinem Benzaldehydcyanohydrin nachgewiesen. Nur das (*R*)-Enantiomer entsteht bzw. wird umgesetzt. Wie schon weiter oben beschrieben, war dieser Umstand nicht vorauszusehen, aufgrund der großen Ähnlichkeit zur MeHNL bzw. HbHNL läge die Annahme einer (*S*)-Selektivität eher auf der Hand.

### Beispiel 7: Strukturuntersuchungen

Auf Basis der Kristallstruktur den HNL aus *Hevea brasiliensis* (PDB Nr. 1QJ4 (ohne Substrat, 1 SC9 (mit Acetoncyanohydrin)) wurde ein Strukturmodell der AtHNL erstellt (s. **Figur 7**).

**Tabelle 4: partielles Substratspektrum der AtHNL (Synthesereaktion)**

| Substrat (Aldehyd oder Keton) | | | Reaktionszeit (h) | Umsatz (%) | ee (R) (%) |
|---|---|---|---|---|---|
| | R¹ | R² | | | |
| Benzaldehyd | H | 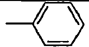 | 2 | > 99,9 | 95 |
| 3-Phenoxybenzaldehyd | H | 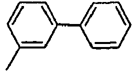 | 61,5 | 73 | 78 |
| 4-Methoxybenzaldehyd | H | 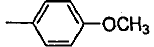 | 22,5 | 22,6 | 0 |
| 4-Chlorbenzaldehyd | H | 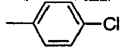 | 7 | 98 | 95 |
| Phenylacetaldehyd | H | 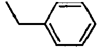 | 22,5 | 90 | 71 |
| Hexanal | H | C₅H₁₁ | 2,5 | 98 | n.b. |
| Heptanal | H | C₆H₁₃ | 2 | > 99,9 | >80% |
| Octanal | H | C₇H₁₅ | 5 | 89 | n.b. |
| Benzylaceton | CH₃ | 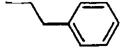 | 22 | 9 | 40 |
| 2-Butanon | CH₃ | C₂H₅ | 22 | >95 | 0 |
| 2-Pentanon | CH₃ | C₃H₇ | 22 | > 95 | 0 |
| 2-Hexanon | CH₃ | C₄H₉ | 22 | n.b. | 0 |
| 2-Heptanon | CH₃ | C₅H₁₁ | 22 | 9 | 9 |
| 2-Octanon | CH₃ | C₆H₁₃ | 22 | 0 | n.b. |

Die Annahme einer α/β-Hydrolasefaltung liegt aufgrund der großen Ähnlichkeit (>65%) zu den bekannten α/β-Hydrolase-ähnlichen HNLs und der konservierten katalytischen Triade (S, D, H) auf der Hand und wird durch das Modell unterstützt. Die Reste der katalytischen Triade liegen auch räumlich gut übereinander. Weiterhin lassen sich aufgrund des Modells einige Ansätze zur Erklärung der Stereoselektivität und des Substratspektrums ableiten. Bei Betrachtung des Substratspektrums fällt auf, dass die getesteten Aldehyde durchweg sehr gut umgesetzt werden, wobei die Aktivität gegenüber Ketonen eher schwach ausfällt. Der Vergleich des Strukturmodells mit der Struktur der HbHNL (mit gebundenem Substrat Acetoncyanohydrin) lässt jedoch den Schluss zu, dass diesem Problem durch eine Punktmutation Abhilfe geschaffen werden kann. Im Vergleich mit der HbHNL-Struktur ist offensichtlich, dass eine Aminosäureseitenkette (Asparagin 12) im Modell weit in die Bindungstasche hineinragt und mit einer Methylgruppe des Substrates kollidiert, während sich in der HbHNL-Kristallstruktur hier eine sterisch weniger ungünstige Aminosäure (Threonin) befindet.

Die Strukturanalyse der erfindungsgemäßen Hydroxynitril-Lyase hat ferner ergeben, dass das Substrat im aktiven Zentrum der AtHNL durch das Alanin an Position 13 (A13), das Phenylalanin an Position 82 (F82, Backbone-NH-Gruppe) und das Asparagin an Position 12 (N12, nachgewiesen durch Austausch) stabilisiert wird. Dem aktiven Zentrum konnten aufgrund ihrer Substratkontakte darüber hinaus noch die Aminosäuren Serin an Position 81 (S81) und Histidin an Position 236 (H236) zugeordnet werden (Um die katalytische Triade zu vervollständigen, gehört hierzu noch die Asparaginsäure an Position 208 (D208)).

Weitere Aminosäurereste in Substratreichweite, d.h. im oder in der Nähe des katalytischen Zentrums bzw. der Substratbindetasche, sind die Folgenden:
M237, A210, L158, F153, L129, M149, Y14, C132, L147, F179, A13, L119, F107 und I211.

Ein erweitertes Substratspektrum der erfindungsgemäßen Hydroxynitril-Lyase ergibt sich aus Tabelle 5.

Erste Versuche zur Stabilisierung der AtHNL bei niedrigeren pH-Werten zeigten, dass Sorbitol und Saccharose bei pH 5 eine Steigerung der Halbwertszeit von 3 Stunden auf mehr als 72 Stunden (bei 200mg /ml Sorbitol) bewirken.

**Tabelle 5: Erweitertes Substratspektrum der AtHNL**

| Substrat | Zeit (h) | *Umsatz* (%) | *ee (R)* (%) | *Nicht enzymatischer Umsatz* (%) (Kontrollreaktion ohne Enzym) |
|---|---|---|---|---|
| 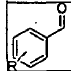 | | | | |
| R=H- **1** | 2 | >99 | >99 | 14 |
| R=*o*-F- **2** | 2 | >99 | 99 | 17 |
| R= *o*-Cl- **3** | 2 | >99 | 99 | 26 |
| R= *o*-Br- **4** | 6 | 99 | 98 | 42 |
| R= *o*-l- **5** | 3 | >99 | >95 | 26 |
| R=*m*-F- **6** | 2 | >99 | >99 | 22 |
| R=*m* -Cl- **7** | 3 | 99 | >99 | 7 |
| R=*m* -Br- **8** | 6 | 99 | 95 | 9 |
| R=*m* -I- **9** | 6 | 98 | 93 | 5 |
| R=*m* -phenoxy **10** | 22 | 83 | >95 | 0 |
| R=*p*-F- **11** | 2 | >99 | >99 | 7 |
| R=*p*-Cl- **12** | 2 | >99 | >99 | 4 |
| R=*p*-Br- **13** | 3 | 99 | >99 | 4 |
| R=*p* -I- **14** | 6 | 99 | 92 | 7 |
| R=*p* -hydroxy- **15** | 3 | 96 | 97 | 3 |
| **R=*p* -methoxy- 16** | 22 | 87 | 68 | 14 |
| 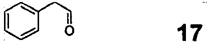 | 22 | 97 | 96 | 97 |
| 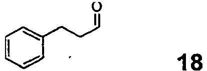 | 22 | 99 | 68 | 97 |
| 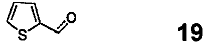 | 6 | 68 | n.d.^{[b]} | 6 |
| 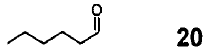 | 6 | 99 | 98 | 78 |
| 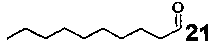 | 22 | 56 | >95 | 0 |
| 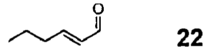 | 3 | 53 | n.d.^{[b]} | 0 |
| 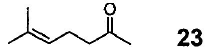 | 22 | 0 | - | 0 |
| 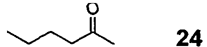 | 6 | 48 | 95 | 2 |
| 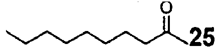 | 22 | 2 | - | 0 |
| 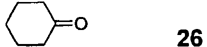 | 3 | 94 | -^{[c]} | 76 |
| 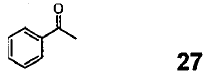 | 22 | 7 | n.d. | 0 |
| 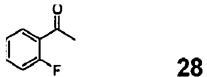 | 22 | 8 | 95 | 0 |
| 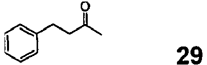 | 3 | 1 | - | 0 |

Zur Unterstützung des Strukturmodells und Verbesserung des erfindungsgemäßen Polypeptids sowie zur Aufklärung des Reaktionsmechanismus hat sich das Einfügen folgender Punktmutationen in SEQ ID Nr. 1 als besonders vorteilhaft herausgestellt:
- Verifizierung der α/β-Hydrolase Faltung: Die als katalytische Triade identifizierten Reste werden gegen andere (sterisch ähnlich, jedoch ohne die entsprechende Funktion) Aminosäuren ausgetauscht, das resultierende Polypeptid weist dann keine Aktivität mehr auf: S81A; D208N; H236F.
- Erweiterung des Substratspektrums auf Ketone: N12T, vorzugsweise als Doppelmutante mit Y14C.
- Umkehr der Enantioselektivität: L129W und/oder Y14C.

Aufgrund begründeter Ansätze zur Verbesserung des erfindungsgemäßen Polypeptids (z.B. bezüglich des Substratspektrums) werden neben dem Wildtypprotein in der Erfindung auch Varianten beansprucht, die durch das Einfügen von einzelnen Punktmutationen in die DNA-Sequenz (rationales Design) sowie durch Methoden der gerichteten Evolution (epPCR, Sättigungsmutagenese usw.) hergestellt worden sind.

### Beispiel 8: Produktion der AtHNL mittels Fed-batch-Fermentation im 15 L-Maßstab

Um die AtHNL im größeren Maßstab herzustellen, kann der exprimierende Stamm in einem Bioreaktor unter Zufütterung fermentiert werden. Als Methode dient ein Standardprotokoll für die Hochzelldichtefermentation von *E. coli* (Korz et al. 1995). Ein 42 L-Fermenter wird mit 100ml Vorkultur in 10 L Medium inokuliert, die Kultur wächst in definiertem Medium für 27h bei 30°C. Die Rate der Zufütterung richtet sich nach der verbrauchten Glucosemenge, so wird für ein optimales Wachstum gesorgt. Nach 27 h wird die Induktion mit IPTG ausgelöst, danach wird das Protein für weitere 16 h exprimiert, anschließend werden die Zellen durch Zentrifugation geerntet. Aus 15 L Endvolumen konnten 1,75 kg Zellen gewonnen werden. Für die anschließende Aufreinigung (s. Beispiel 4) werden 20 g Zellen eingesetzt, in Aufschlusspuffer resuspendiert, durch Ultraschall aufgeschlossen, zentrifugiert und der Überstand als Rohextrakt verwendet. Dieser Rohextrakt (Endvolumen ca. 30 ml) hat eine volumetrische Aktivität von ca. 800 U/ml und eine spezifische Aktivität von 13,3 U/mg. Nach Reinigung über eine Q-Sepharose-Säule und anschließender Entsalzung (s. Beispiel 4) sowie der Lyophilisierung ergibt sich eine Aktivität von 2,5 U/mg Lyophilisat und eine spezifische Aktivität von etwa 40 U/mg Protein. Ausgehend von 20 g Zellen können so etwa 6 g Lyophilisat gewonnen werden, was einer Gesamtaktivität von ca. 15 kU entspricht. Aus einer Hochzelldichtefermentation mit 15 L Endvolumen können also insgesamt 525 g Lyophilisat mit einer Gesamtaktivität von 1300 kUnits gewonnen werden.

Dieses Beispiel zeigt, dass das erfindungsgemäße Polypeptid überraschender Weise problemlos auch in großen Mengen produziert werden kann. Dabei entspricht die Ausbeute im Fermenter derjenigen, die im Labormaßstab erreicht wurde.

### Literatur:

Albrecht J, Jansen I, Kula MR (1993) Improved Purification of an (R)-Oxynitrilase from Linum-Usitatissimum (Flax) and Investigation of the Substrate Range. Biotechnol Appi Biochem 17: 191-203
Andexer J, Guterl JK, Pohl M, Eggert T (2006) A high-throughput screening assay for hydroxynitrile lyase activity: Chem Commun 40: 4201-4203
Bray EA (2002) Classification of genes differentially expressed during water-deficit stress in Arabidopsis thaliana: an analysis using microarray and differential expression data. Ann Bot 89: 803-11
Bauer M, Griengl H, Steiner W (1999) Kinetic studies on the enzyme (S)-hydroxynitrile lyase from hevea brasiliensis using initial rate methods and progress curve analysis. Biotechnol Bioeng 62: 20-29
Bühler H, Effenberger F, Förster S, Roos J, Wajant H (2003) Substrate specificity of mutants of the hydroxynitrile lyase from Manihot esculenta. Chembiochem 4:211-216
Fechter MH, Griengl H (2004) Hydroxynitrile lyases: Biological sources and application as biocatalysts. Food Technol Biotechnol 42: 287-294
Gaisberger RP, Fechter MH, Griengl H (2004) The first hydroxynitrile lyase catalysed cyanohydrin formation in ionic liquids. Tetrahedron Asymmetry 15: 2959-2963
Glieder A, Weis R, Skranc W, Poechlauer P, Dreveny I, Majer S, Wubbolts M, Schwab H, Gruber K (2003) Comprehensive step-by-step engineering of an (R)-hydroxynitrile lyase for large-scale asymmetric synthesis. Angew Chem Int Ed Engl 42:4815-4818
Hasslacher M, Kratky C, Griengl H, Schwab H, Kohlwein SD (1997) Hydroxynitrile lyase from Hevea brasiliensis: molecular characterization and mechanism of enzyme catalysis. Proteins 27: 438-449
Korz DJ, Rinas U, Hellmuth K, Sanders EA und Deckwer W-D (1995) Simple fed-batch technique for high cell density cultivation of Escherichia coli. J Biotechnol 39 (1). 59-65
Lee F, Mulligan R, Berg P, Ringold G (1981) Glucocorticoids regulate expression of dihydrofolate reductase cDNA in mouse mammary tumour virus chimaeric plasmids. Nature 294: 228-232
Ollis DL, Cheah E, Cygler M, Dijkstra B, Frolow F, Franken SM, Harel M, Remington SJ, Silman I, Schrag J (1992) The alpha/beta hydrolase fold. Protein Eng 5:197-211 1
Rosenthaler L (1908) Durch Enzyme bewirkte asymmetrische Synthesen. Biochem Z 14:238-253
Schmidt M, Griengl H (1999) Oxynitrilases: From cyanogenesis to asymmetric synthesis. Biocatalysis - from Discovery to Application 200: 193-226
Sharma M, Sharma NN, Bhalla TC (2005) Hydroxynitrile lyases: At the interface of biology and chemistry. Enzyme Microb Technol 37:279-294
Wäspi U, Misteli B, Hasslacher M, Jandrositz A, Kohlwein SD, Schwab H, Dudier R (1998) The defense-related rice gene Pir7b encodes an alpha/beta hydrolase fold protein exhibiting esterase activity towards naphthol AS-esters. Eur J Biochem 254:32-37

## Patentansprüche

1. Polypeptid, isoliert aus Pflanzen der Familie Brassicaceae, insbesondere der Gattung *Arabidopsis,* bevorzugt der Art *Arabidopsis thaliana,* mit der Aminosäuresequenz gemäß SEQ ID NR. 1, das zumindest die Aktivität einer Hydroxynitril - Lyase aufweist und (R) - selektiv ist, **gekennzeichnet durch** einen oder mehrere der folgenden Aminosäure-Austausch(e) in Bezug auf SEQ ID NR. 1:
a) Asparagin an Position 12 gegen Threonin (N12T) oder Alanin (N12A);
b) Tyrosin an Position 14 gegen Cystein (Y14C) oder Alanin (Y14A);
c) Leucin an Position 129 gegen Tryptophan (L129W),
wobei die Austausche Y14C und L129W eine Umkehr der Enantioselektivität bewirken.

2. Polypeptid nach Anspruch 1, das zumindest die Synthese von chiralen Cyanohydrinen aus Aldehyden oder Ketonen und Blausäure katalysiert.

3. Protein, insbesondere Fusionsprotein, das mindestens ein Polypeptid nach einem der Ansprüche 1 oder 2 umfasst.

4. Isoliertes Nukleinsäuremolekül, das mindestens eine Nukleotidsequenz zur Synthese mindestens eines Polypeptids umfasst, wobei die Nukleotidsequenz ausgewählt ist aus der Gruppe bestehend aus:
a) Nukleotidsequenz, welche für ein Polypeptid nach einem der Ansprüche 1 oder 2 kodiert;
b) Nukleotidsequenz, welche sich von der Nukleotidsequenz gemäß a) durch den Austausch zumindest eines Codons gegen ein synonymes Codon unterscheidet;
c) Nukleotidsequenz, welche dem komplementären Strang der Nukleotidsequenz gemäß a) entspricht.

5. Polypeptid, hergestellt durch Expression eines Gens, welches das Nukleinsäuremolekül nach Anspruch 4 umfasst.

6. Vektor zur Synthese eines Polypeptids in einer geeigneten Zelle, welcher das Nukleinsäuremolekül nach Anspruch 4 in exprimierbarer Form umfasst.

7. Zelle, welche das Nukleinsäuremolekül nach Anspruch 4 und/oder den Vektor nach Anspruch 6 enthält.

8. Verwendung des Polypeptids nach Anspruch 1, 2 oder 5, oder des Proteins nach Anspruch 3, oder der Zelle nach Anspruch 7, zur Herstellung von chiralen Cyanohydrinen.

9. Verwendung eines Polypeptids mit der Aminosäuresequenz gemäß SEQ ID NR. 1, das aus Pflanzen der Familie Brassicaceae, insbesondere der Gattung *Arabidopsis,* bevorzugt der Art *Arabidopsis thaliana,* isoliert wurde und das zumindest die Aktivität einer Hydroxynitril - Lyase aufweist, zur (R)-selektiven Herstellung von chiralen Cyanohydrinen.

10. Verfahren zur Synthese von chiralen Cyanohydrinen aus mindestens einer Carbonylverbindung und Blausäure, bei dem die Reaktion in Anwesenheit des Polypeptids nach einem der Ansprüche 1, 2 oder 5, oder des Proteins nach Anspruch 3, oder der Zelle nach Anspruch 7, durchgeführt wird.

11. Verfahren zur (*R*)-selektiven Synthese von chiralen Cyanohydrinen aus mindestens einer Carbonylverbindung und Blausäure, bei dem die Reaktion in Anwesenheit eines Polypeptids mit der Aminosäuresequenz gemäß SEQ ID NR. 1, das aus Pflanzen der Familie Brassicaceae, insbesondere der Gattung *Arabidopsis,* bevorzugt der Art *Arabidopsis thaliana,* isoliert wurde und das zumindest die Aktivität einer Hydroxynitril - Lyase aufweist, durchgeführt wird.

12. Verfahren zur Spaltung von Cyanohydrinen in mindestens eine Carbonylverbindung und Blausäure, bei dem die Reaktion in Anwesenheit des Polypeptids nach einem der Ansprüche 1, 2 oder 5, oder des Proteins nach Anspruch 3, oder der Zelle nach Anspruch 7, durchgeführt wird.

13. Verfahren zur (*R*)-selektiven Spaltung von Cyanohydrinen in mindestens eine Carbonylverbindung und Blausäure, bei dem die Reaktion in Anwesenheit eines Polypeptids mit der Aminosäuresequenz gemäß SEQ ID NR. 1, das aus Pflanzen der Familie Brassicaceae, insbesondere der Gattung *Arabidopsis,* bevorzugt der Art *Arabidopsis thaliana,* isoliert wurde und das zumindest die Aktivität einer Hydroxynitril - Lyase aufweist, durchgeführt wird.

## Claims

1. Polypeptide isolated from plants of family Brassicaceae, in particular genus *Arabidopsis,* preferably species *Arabidopsis thaliana,* having the amino acid sequence of SEQ ID NO. 1, exhibiting at least the activity of a hydroxynitrile lyase and being (R) - selective, **characterized by** one or more of the amino acid replacements in respect of SEQ ID NO. 1 set forth hereunder:
a) Asparagine at position 12 by threonine (N12T) or alanine (N12A);
b) Tyrosine at position 14 by cysteine (Y14C) or alanine (Y14A);
c) Leucine at position 129 by tryptophan (L129W),
wherein the replacements Y14C and L129W cause a change of enantioselectivity.

2. Polypeptide according to claim 1 catalyzing at least the synthesis of chiral cyanohydrines from aldehydes or ketones and hydrocyanic acid.

3. Protein, in particular fusion protein, comprising at least one polypeptide according to any one of claims 1 or 2.

4. Isolated nucleic acid molecule comprising at least one nucleotide sequence for synthesizing at least one polypeptide, wherein the nucleotide sequence is selected from the group consisting of:
a) Nucleotide sequence coding a polypeptide according to any one of claims 1 or 2;
b) Nucleotide sequence that differs from the nucleotide sequence according to a) by the replacement of at least one codon by a synonymous codon;
c) Nucleotide sequence corresponding to the complementary strand of the nucleotide sequence according to a).

5. Polypeptide synthesized by expression of a gene comprising the nucleic acid molecule according to claim 4.

6. Vector for synthesizing a polypeptide in a suitable cell, which comprises the nucleic acid molecule according to claim 4 in expressible form.

7. Cell including the nucleic acid molecule according to claim 4 and/or the vector according to claim 6.

8. Use of the polypeptide according to claim 1, 2, or 5 or the protein according to claim 3 or the cell according to claim 7 for producing chiral cyanohydrines.

9. Use of a polypeptide having the amino acid sequence of SEQ ID NO. 1, being isolated from plants of family Brassicaceae, in particular genus *Arabidopsis,* preferably species *Arabidopsis thaliana,* and exhibiting at least the activity of a hydroxynitrile lyase for (R)-selective production of chiral cyanohydrines.

10. Method for synthesizing chiral cyanohydrines from at least one carbonyl compound and hydrocyanic acid, wherein the reaction is conducted in the presence of the polypeptide according to any one of claims 1, 2, or 5 or the protein according to claim 3 or the cell according to claim 7.

11. Method for (*R*)-selective synthesis of chiral cyanohydrines from at least one carbonyl compound and hydrocyanic acid, wherein the reaction is conducted in the presence of a polypeptide having the amino acid sequence of SEQ ID NO. 1, being isolated from plants of family Brassicaceae, in particular genus *Arabidopsis,* preferably species *Arabidopsis thaliana,* and exhibiting the activity of a hydroxynitrile lyase.

12. Method for cleaving cyanohydrines into at least one carbonyl compound and hydrocyanic acid, wherein the reaction is conducted in the presence of the polypeptide according to any one of claims 1, 2, or 5 or the protein according to claim 3 or the cell according to claim 7.

13. Method for (*R*)-selective cleavage of cyanohydrines into at least one carbonyl compound and hydrocyanic acid, wherein the reaction is conducted in the presence of a polypeptide having the amino acid sequence of SEQ ID NO. 1, being isolated from plants of family Brassicaceae, in particular genus *Arabidopsis,* preferably species *Arabidopsis thaliana,* and exhibiting the activity of a hydroxynitrile lyase.

## Revendications

1. Polypeptide, isolé à partir de plantes appartenant à la famille des Brassicaceae, notamment au genre *Arabidopsis,* de préférence à l'espèce *Arabidopsis thaliana,* lequel a la séquence d'acides aminés selon SEQ ID N°1 et lequel présente au moins l'activité d'une hydroxynitrile lyase et une sélectivité (*R*), **caractérisé par** un ou plusieurs des remplacements d'acides aminés suivants, par rapport à la SEQ ID N° 1 :
a) l'asparagine à la position 12 par une thréonine (N12T) ou une alanine (N12A) ;
b) la tyrosine à la position 14 par une cystéine (Y14C) ou une alanine (Y14A) ;
c) la leucine à la position 129 par un tryptophane (L129W),
les remplacements Y14C et L129W provoquant une inversion de l'énantiosélectivité.

2. Polypeptide selon la revendication 1, catalysant au moins la synthèse de cyanohydrines chirales à partir d'aldéhydes ou de cétones et d'acide cyanhydrique.

3. Protéine, notamment protéine de fusion, comprenant au moins un polypeptide selon l'une des revendications 1 ou 2.

4. Molécule d'acide nucléique isolée, comprenant au moins une séquence de nucléotides destinée à la synthèse d'au moins un polypeptide, ladite séquence de nucléotides étant choisie dans le groupe constitué :
a) d'une séquence de nucléotides codant pour un polypeptide selon l'une des revendications 1 ou 2 ;
b) d'une séquence de nucléotides se distinguant de la séquence de nucléotides selon a) en ce qu'au moins un codon est remplacé par un codon synonyme ;
c) d'une séquence de nucléotides correspondant au brin complémentaire à la séquence de nucléotides selon a).

5. Polypeptide, obtenu par expression d'un gène comprenant la molécule d'acide nucléique selon la revendication 4.

6. Vecteur destiné à la synthèse d'un polypeptide dans une cellule appropriée, comprenant la molécule d'acide nucléique selon la revendication 4 sous une forme apte à être exprimée.

7. Cellule contenant la molécule d'acide nucléique selon la revendication 4 et/ou le vecteur selon la revendication 6.

8. Utilisation du polypeptide selon les revendications 1, 2 ou 5, ou de la protéine selon la revendication 3, ou de la cellule selon la revendication 7, pour obtenir des cyanohydrines chirales.

9. Utilisation d'un polypeptide ayant la séquence d'acides aminés selon la SEQ ID N° 1, lequel a été isolé à partir de plantes appartenant à la famille des Brassicaceae, notamment au genre *Arabidopsis,* de préférence à l'espèce *Arabidopsis thaliana,* et lequel présente au moins l'activité d'une hydroxynitrile lyase, pour obtenir des cyanohydrines chirales avec une sélectivité *(R)*.

10. Procédé destiné à la synthèse de cyanohydrines chirales à partir d'au moins un composé carbonyle et d'acide cyanhydrique, dans lequel la réaction est réalisée en présence du polypeptide selon l'une des revendications 1, 2 ou 5, ou de la protéine selon la revendication 3, ou de la cellule selon la revendication 7.

11. Procédé destiné à la synthèse de cyanohydrines chirales à partir d'au moins un composé carbonyle et d'acide cyanhydrique avec une sélectivité *(R)*, dans lequel la réaction est réalisée en présence d'un polypeptide qui a la séquence d'acides aminés selon SEQ ID N° 1 et qui a été isolé à partir de plantes appartenant à la famille des Brassicaceae, notamment au genre Arabidopsis, de préférence à l'espèce *Arabidopsis thaliana,* et qui présente au moins l'activité d'une hydroxynitrile lyase.

12. Procédé destiné au clivage de cyanohydrines pour obtenir au moins un composé carbonyle et de l'acide cyanhydrique, dans lequel la réaction est réalisée en présence du polypeptide selon l'une des revendications 1, 2 ou 5, ou de la protéine selon la revendication 3, ou de la cellule selon la revendication 7.

13. Procédé destiné au clivage de cyanohydrines pour obtenir au moins un composé carbonyle et de l'acide cyanhydrique avec une sélectivité *(R)*, dans lequel la réaction est réalisée en présence d'un polypeptide qui a la séquence d'acides aminés selon SEQ ID N° 1 et qui a été isolé à partir de plantes appartenant à la famille des Brassicaceae, notamment au genre *Arabidopsis,* de préférence à l'espèce *Arabidopsis thaliana,* et qui présente au moins l'activité d'une hydroxynitrile lyase.
